(19) Europäisches Patentamt
European Patent Office
Office européen des brevets

(11) Publication number: **0 218 543 B1**

# (12) EUROPEAN PATENT SPECIFICATION

(45) Date of publication of patent specification: **28.08.91**

(51) Int. Cl.⁵: **C07D 213/64**, C07D 213/70, C07D 239/34, C07D 239/38, C07D 277/14, C07D 277/16, C07D 277/34, C07D 277/36, A01N 43/40, A01N 43/54, A01N 43/78

(21) Application number: **86810302.9**

(22) Date of filing: **09.07.86**

The file contains technical information submitted after the application was filed and not included in this specification

## (54) Nitrogen-containing heterocyclic compounds.

(30) Priority: **18.07.85 US 756873**
**28.04.86 US 856563**

(43) Date of publication of application:
**15.04.87 Bulletin 87/16**

(45) Publication of the grant of the patent:
**28.08.91 Bulletin 91/35**

(84) Designated Contracting States:
**AT BE CH DE FR GB IT LI LU NL SE**

(56) References cited:
**EP-A- 0 128 648**
**US-A- 4 460 588**

(73) Proprietor: **SANDOZ AG**
**Lichtstrasse 35**
**CH-4002 Basel(CH)**
(84) Designated Contracting States:
**BE CH FR GB IT LI LU NL SE**

Proprietor: **SANDOZ-PATENT-GMBH**
**Humboldtstrasse 3**
**W-7850 Lörrach(DE)**
(84) Designated Contracting States:
**DE**

Proprietor: **SANDOZ-ERFINDUNGEN Verwaltungsgesellschaft m.b.H.**
**Brunner Strasse 59**
**A-1235 Wien(AT)**
(84) Designated Contracting States:
**AT**

(72) Inventor: **Henrick, Clive Arthur**
**3177 Manchester Ct.**
**Palo Alto, CA 94303(US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

This invention relates to novel nitrogen-containing heterocyclio compounds, their synthesis, the use of the novel compounds for the control of pests and to pest controlling compositions comprising such novel compounds.

EPA 0 128 648 discloses nitrogen-containing heterocyclic compounds that are useful for the control of insects. Included within the scope of the disclosure are certain substituted (phenoxyphenoxy)alkoxy pyridine compounds.

EPA 0 203 798 is also directed to nitrogen-containing heterooyolic compounds that are useful for the control of insects, and includes within its scope certain substituted (alkoxyphenoxy)alkoxy pyridine compounds.

The novel nitrogen heterocyclic compounds of this invention are represented by the following formula (A):

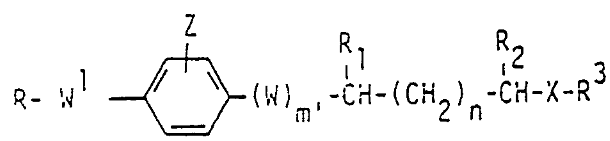

(A)

wherein m' is zero or one;
n is zero, one or two;
W is oxygen, sulfur, $NR^4$, or carbonyl;
$W^1$ is oxygen, sulfur, $NR^4$, carbonyl, sulfinyl or sulfonyl;
X is oxygen or sulfur;
Z is hydrogen, $C_{1-8}$alkyl, $C_{1-8}$haloalkyl or halogen;
R is $C_{3-8}$alkyl, $C_{2-8}$alkenyl, $C_{2-8}$alkynyl, $C_{1-8}$haloalkyl, $C_{2-8}$haloalkenyl, $C_{2-8}$haloalkynyl, $C_{2-10}$alkylthioalkyl, $C_{3-8}$cycloalkyl, $C_{3-8}$halocycloalkyl, $C_{4-12}$cycloalkylalkyl or heterocycloalkyl;
each of $R^1$ and $R^4$ is independently hydrogen or $C_{1-8}$alkyl;
$R^2$ is hydrogen, $C_{1-8}$alkyl or halogen; and
either $R^3$ is an aromatic nitrogen-containing heteroring selected from pyridyl, 3-pyridazinyl , 2-pyrimidinyl, pyrazinyl, triazinyl or 2-thiazolyl which aromatic nitrogen-containing heteroring may be unsubstituted or substituted by one or more substituents selected from halogen, $C_{1-8}$alkyl, $C_{1-8}$haloalkyl, $C_{1-8}$alkoxy, $C_{1-8}$alkylthio and $NO_2$,
or is a group ($G_1$):

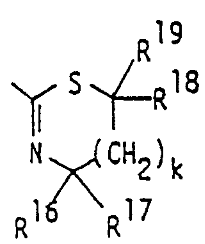

($G_1$)

in which k is zero or one; and
each of $R^{16}$, $R^{17}$, $R^{18}$, $R^{19}$, is independently hydrogen or methyl,
provided that
a) when
m' is one,
X, $W^1$ and W are oxygen,
$R^3$ is 2-pyridyl,
n is O,
and Z, $R^1$ and $R^2$ are H,
then R is not 2-methylpropyl, isopentyl, ethyl, n-butyl, n-propyl, n-pentyl, 2-methylbutyl, 3,3-dimethylbutyl, 2,2-dimethylpropyl, 2-methyl-2-propenyl or 2-propenyl;

or b) when
R is isopentyl
m' is one,
$W^1$ and W are oxygen,
n is O,
X is oxygen,
and Z, $R^1$ and $R^2$ are H,
then $R^3$ is not 2-pyrimidinyl or 2-thiazolyl;
or c) when
R is isopentyl
m' is one,
$W^1$ and W are oxygen,
n is O
x is oxygen,
Z and $R^1$ are H,
and $R^2$ is methyl,
then $R^3$ is not 2-pyridyl, 2-pyrimidinyl or 2-thiazolyl;
or d) when
R is isopentyl
m' is one,
$W^1$ and W are oxygen,
n is O,
X is oxygen,
Z and $R^2$ are H,
and $R^1$ is methyl
then $R^3$ is not 2-pyrimidinyl, 2-pyridyl or 2-thiazolyl;
or e) when
R is isopentyl
m' is one,
$W^1$ and W are oxygen,
n is O,
X is sulfur,
and Z, $R^1$ and $R^2$ are H,
then $R^3$ is not 2-thiazolin-2-yl, 2-pyridyl or 2-pyrimidinyl;
or f) when
R is isopentyl
m' is one,
$W^1$ and W are oxygen,
n is O,
X is oxygen,
Z is Cl in ortho position of $RW^1$,
and $R^1$ and $R^2$ are H,
then $R^3$ is not 2-pyridyl or 2-thiazolyl;
or g) when
m' is one,
X, $W^1$ and W are oxygen,
$R^3$is 2-thiazolyl,
n is O,
and Z, $R^1$ and $R^2$ are H,
then R is not isobutyl.

The terms $C_{1-8}$haloalkyl, $C_{2-8}$haloalkenyl, $C_{2-8}$haloalkynyl and $C_{3-8}$halocycloalkyl refer to $C_{1-8}$alkyl, $C_{2-8}$alkenyl, $C_{2-8}$alkynyl and $C_3$-cycloalkyl resp. substituted with one to six halogen, particularly to 1 to 3 halogen atoms.

Any halogen is preferably Cl or F.

The terms alkenyl and alkynyl when used herein refer to such hydrocarbyl groups having 1 or 2, preferably 1, ethylenic bonds or 1 or 2, preferably 1, acetylenic bonds resp.

Where R is $C_{3-8}$alkyl, $C_{2-8}$alkenyl or $C_{2-8}$alkynyl it refers particularly to an alkyl, alkenyl or alkynyl group having 3-6, more particularly 4 or 5S carbon atoms; such groups are preferably branched.

R is preferably $C_{3-8}$alkyl or $C_{2-8}$alkenyl. A particularly preferred alkyl significance of R is 2-butyl. A particularly preferred alkenyl significance of R is 3-methyl-2-butenyl (i.e. $(CH_3)_2C=CH\text{-}cH_2$).

The term "heterocycloalkyl" refers to a saturated or unsaturated heteroring comprising 2 to 6 carbon ring atoms and 1 to 3 heteroring atoms selected from nitrogen, oxygen or sulfur.

Where $R^3$ is substituted pyridyl, it may bear one to four substituents. Where $R^3$ is substituted 3-pyridazinyl, 2-pyrimidinyl, pyrazinyl or triazinyl it is conveniently mono- or disubstituted.

Where $R^3$ is substituted aromatic nitrogen-containing heteroring it is preferably monosubstituted, particularly by halogen.

Preferred $R^3$ significances are 2-pyridyl, 2-pyrimidinyl, 2-thiazolyl, 2-thiazolin-2-yl. (2-Thiazolyl refers to 1,3-thiazol-2-yl).

m' is preferably 1.

Z is preferably H.

W is preferably oxygen.

$W^1$ is preferably O or S.

n is preferably zero.

$R^1$ is preferably H or $C_{1-5}$alkyl, more preferably H or $CH_3$. $R^2$ is preferably H, $C_{1-5}$alkyl or halogen, more preferably H, $CH_3$ or halogen. Preferably at least one of $R^1$ and $R^2$ is H.

Where Z is $C_{1-8}$alkyl or $C_{1-8}$haloalkyl and/or $R^4$ is $C_{1-8}$alkyl, such groups comprise conveniently 1 to 5 carbon atoms.

Where $R^3$ comprises $C_{1-8}$alkyl, $C_{1-8}$haloalkyl, $C_{1-8}$alkoxy and/or $C_{1-8}$-alkylthio substituents, these have conveniently 1 to 5 carbon atoms.

The compounds of formula (A) are obtained by etherifying a compound of formula (I)

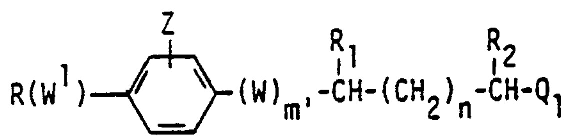 (I)

wherein R, $R^1$, $R^2$, $R^1$ , W, Z, m' and n are as defined above,
and $Q_1$ is OH, SH or a leaving group capable of being split off under the reaction conditions
with a compound of formula (II)

$Q_2$ - $R^3$ (II)

wherein $R^3$ is as defined above
and $Q_2$ is a leaving group capable of being split off under the reaction conditions where $Q_1$ is OH or SH, or is OH or SH where $Q_1$ is a leaving group,
followed, where desired, by halogenation of the thus obtained compounds of formula (A), wherein $R^2$ is H, X is S and W and $W^1$ are O to compounds of formula (A) wherein $R^2$ is halogen, X is S and W and $W^1$ are O.

The reaction of compounds of formula (I) with compounds of formula (II) may be effected under the conditions known for the preparation of ethers or thioethers.

The reaction is conveniently carried out in an organic solvent which is inert under the reaction conditions, such as N-methylpyrrolidone, an ether such as tetrahydrofuran, an amide of a carboxylic acid such as dimethylformamide. A suitable reaction temperature is between 0° and 140°C, preferably at between 10° and 110°C. it is advantageous to effect the reaction in the presence of a base such as potassium carbonate or sodium hydroxide. Alternatively, the alcohol or thiol component may also be first converted in its salt form, e.g. by reaction with an alkali metal hydride such as NaH, and then further reacted.

Examples of suitable leaving groups ($Q_1$ or $Q_2$) are Cl, Br, I, the mesyloxy or tosyloxy group.

The optional halogenation of compounds of formula (A) wherein $R^2$ is hydrogen, X is S and W and $W^1$ are O can be carried out with a suitable halogenating agent such as N-halosuccinimide, preferably in an equivalent or greater amount. The rection may be effected in a solvent which is inert under the reaction conditions, e.g. in a chlorinated hydrocarbon such as carbon tetrachloride, 1,1-dichloroethane or methylene chloride, and if desired in the presence of a radical initiator.

The compounds of formula (A) may be recovered from the reaction mixture in which they are formed by working up by established procedures.

The compounds of the formula (A) can have one or more asymmetric centers and/or geometric

isomers. The present invention includes each of the stereo isomers and the mixtures of stereo isomers thereof. In the examples hereinafter, unless otherwise specified, the compound is a mixture of stereo isomers.

The starting materials and reagents employed in the processes described above are either known or, insofar as they are not known, may be produced in analogous manner to the processes described herein or to known processes.

The compounds of formula (A) show interesting activity against a wide range of pests, such as fleas (Ctenocephalides felis), ticks, flies (Musca domestica), cockroaches (Blattella germanica), eggs of Spodoptera species etc. In view of their interesting activity the compounds can be effective control agents for insects of, for example, the orders Lepidoptera, Hemiptera, Homoptera, Coleoptera, Diptera, Orthoptera, and Siphonaptera, and for mites and ticks of the class Acari, including mites of the families Tetranychidae or Tarsonemidae and ticks of the families Argasidae and Ixodidae.

The optimum usage of a compound of formula (A) is readily determined by a person of ordinary skill in the art using routine laboratory testing. Usually satisfactory results are obtained with a test amount of the order of 0.1 ug to 100 $\mu$g per insect, mite or tick, depending on the mode and conditions of application as well as on the pest involved.

They are preferably applied to the immature insect, namely during the egg, embryo, larval or prepupal stage, in view of their effect on metamorphosis and otherwise abnormal development leading to death or inability to reproduct. For many of the compounds of formula (A) the effective application rate is equivalent to or below that known for commercially available IGR compounds such as methoprene, hydroprene etc. In view of their interesting pest controlling effect the compounds of formula (A) are indicated for use in i.a. crop protection, forest protection, horticulture protection, stored food and tobacco protection, cattle and pest protection, against cockroaches, ants, termites, fleas, fruit tree leafrollers, whiteflies, aphids, scales, leafminers, ectoparasites etc.

Application of a compound of formula (A) is made according to conventional procedures, involving the application to the pest (insects, mites, ticks) or their locus of a pest controlling amount of a compound of formula (A). The amount to be applied will depend on the target, the mode and conditions of application and the like. Thus, for example for crop protection, satisfactory results will, in general, be obtained with application rates of from 0.01 to 1 kg of compound of formula (A) per hectare.

The compounds of formula (A) are conveniently employed in pest controlling composition form in association with a diluent.

Such compositions also form part of the present invention. They may contain, aside from a compound of formula (A) as active agent, other active agents, such as insecticides (e.g. synthetic pyrethroids, carbamates, phosphates), insect growth regulators or insect attractants. They may be employed in either solid or liquid forms e.g. in the form of a wettable powder or an emulsifiable concentrate incorporating conventional diluents. Such compositions may be produced in conventional manner, e.g. by mixing the active ingredient with a diluent and optionally other formulating ingredients such as surfactants.

The term diluents as used herein means any liquid or solid agriculturally acceptable material which may be added to the active constituent to bring it in an easier or improved applicable form, respectively to a usable or desirable strength of activity. It can for example be talc, kaolin, diatomaceous earth, xylene, or water.

Particularly formulations to be applied in spraying forms such as water dispersible concentrates or wettable powders may contain surfactants such as wetting and dispersing agents, e.g. the condensation product of formaldehyde with naphthalene sulphonates, an alkylarylsulphonate, a lignin sulphonate, a fatty alkyl sulphate, an ethoxylated alkylphenol and an ethoxylated fatty alcohol.

In general, the formulations include from 0.01 to 90% by weight of active agent, from 0 to 20% by weight of agriculturally acceptable surfactant and 99.99 to 10% by weight (solid or liquid) diluent(s), the active agent consisting either of at least one compound of formula (A) or mixtures thereof with other active agents. Concentrate forms of compositions generally contain between about 2 and 90%, preferably between about 5 and 65% by weight of active agent. Application forms of formulations may for example contain from 0.01 to 25% by weight, preferably from 0.01 to 5% by weight of active agent or more diluted forms thereof: they include sprays, foggers, baits, encapsulated form, cyclodextrin inclusion complexes, pet collars, ear tags and the like.

The following examples are provided to illustrate the practice of the present invention. Temperatures are given in degrees centigrade and parts and percentages are by weight. RT means room temperature.

**Composition Example**

**Emulsifiable Concentrate**

65 Parts of a compound of formula (A), e.g. compound 2 hereinafter given, are mixed with 8 parts of an emulsifier (e.g. 4 parts of Atlox 3404 F and 4 parts of Atlox 848, which are mixtures of anionic and non-ionic emulsifiers of ICI America) and 27 parts of an organic solvent (e.g. xylene or Tenneco 500-100, which is a mixture of trimethyl benzene and xylene solvents of Tenneco Corporation) are thoroughly mixed until a homogeneous solution is obtained.

**EXAMPLE 1 :**

To a mixture of sodium hydride (0.08 g) in 5 ml of tetrahydrofuran (THF) and 2 ml of hexamethylphosphoramide (HMPA), cooled in an ice bath, is added, dropwise, 2-methyl 2-[4-(1-methylpropoxy)-phenoxy]ethanol (0.63g). The mixture is stirred at RT for 2 hours, after which a solution of 2-chloro pyridine (0.38 g) in 5 ml of THF is added and the mixture is stirred at 60° for 3 hours. The THF is then removed by rotoevaporation and the residue is purified by column chromatography on silica gel to give the product, 2-{2-methyl-2-[4-(1-methylpropoxy)phenoxy]ethoxy}pyridine, MS m/e 302 ($M^+$)-(Compound 1 of Table I).

**EXAMPLE 2 :**

To a mixture of sodium hydride (0.12 g) in 5 ml of THF and 4 ml of HMPA, cooled in an ice bath, is added, dropwise, a solution of 2-[4-(1-methylpropoxy)phenoxy]ethanol (1.00 g) in 5 ml of THF. The mixture is stirred at RT for 2 hours, after which 2,6-difluoropyridine (0.65 g) is added and the mixture is stirred at RT overnight. The THF is removed by rotoevaporation and the product is isolated by column chromatography on silica gel to give 6-fluoro-2-{2-[4-(1-methylpropoxy)phenoxy]ethoxy}pyridine, MS m/e 306 ($M^+$). (Compound 7 of the Table I).

**EXAMPLE 3 :**

To sodium hydride (0.17 g, 7.14 mmol) in 10 of dimethylformamide (DMF) is added, at RT, 2-[4-(1-methylpropoxy)phenoxy]ethanol (1.50 g, 7.14 mmol) in 3 ml of DMF. The mixture is heated to 50° and stirred for 1 hour. 2-Chloropyrimidine (0.94 g, 8.21 mmol) is then added, at 55°, and the mixture is stirred for 1 hour and then cooled to RT. The reaction mixture is poured into ice water and extracted with ether. The combined organic layers are washed with water until neutral and with brine, dried and filtered and the solvent is removed to give, following purification of the residue by preparative thin layer chromatography (prep. TLC), 2-{2-[4-(1-methylpropoxy)phenoxy]ethoxy}pyrimidine, MS m/e 289 ($M^+$ + H), (Compound 12 of Table I).

**EXAMPLE 4 :**

To sodium hydride (0.35 g, 14.6 mmol) in 20 ml of DMF at RT is slowly added 2-mercaptopyrimidine (1.64 g, 14.6 mmol) in 30 ml of DMF, keeping the temperature of the reaction at 30° or below. After anion formation is complete 2-[4-(1-methylpropoxy)phenoxyethyl bromide (4.0 g, 14.6 mmol) is slowly added, with cooling to keep the reaction temperature at 15-20°. The mixture is then stirred at RT for 18 hours, after which it is poured into water and extracted with ether. The combined organic layers are washed with water and with brine, dried and filtered and the solvent is removed to give, after purification by prep. TLC, 2-{2-[4-(1-methylpropoxy) phenoxy]ethylthio} pyrimidine, MS m/e 305 ($M^+$ + H). (Compound 20 of Table I).

**EXAMPLE 5 :**

A mixture of 2-(4-isopropoxyphenoxy)ethanol (3.50 g), mesyl chloride (2.20 g) and triethylamine (2.0 g) in 20 ml of methylene chloride is stirred at RT for 3 hours. It is then poured into water and extracted with methylene chloride. The solvent is removed from the combined organic layers by rotoevaporation, and the residue is purified by column chromatography to give 2-(4-isopropoxyphenoxy)ethyl methanesulfonate.

Following the procedure of Example 4, 2-mercapto-2-thiazoline (0.77g is treated with sodium hydride (0.32 g), and the resulting sodium salt is reacted with the above methanesulfonate (1.50 g) to give 2-[2-(4-isopropoxyphenoxy)ethylthio]-2-thiazoline (Compound 61 of Table I).

**EXAMPLE 6 :**

N-chlorosuccinimide (1.05 g, 7.9 mmol) is added in portions to 2-{2-[4-(1-methylpropoxy)phenoxy)-phenoxy]ethylthio}-pyrimidine (2.0 g, 6.6 mmol) in carbon tetrachloride at 5°. The mixture is warmed to RT and stirred for 18 hours, followed by heating to 60° for 32 hours. The reaction mixture is filtered and the solvent is removed to give, following purification by chromatography, 2-{1-chloro-2-[4-(1-methylpropoxy)-phenoxy]ethylthio}pyrimidine MS m/e 338 ($M^+$) (Compound 44 of Table I).

The following compounds of formula (A) are obtained according to the processes disclosed herein, employing appropriate starting materials (see Table I).

TABLE I : Compounds of formula (A) wherein $(W)_{m'}$ is 0

| Cpd. | $R\text{-}W^1$ | $CH(R_1)\text{-}(CH_2)_n\text{-}CHR_2$ | $X\text{-}R_3$ |
|---|---|---|---|
| 1 | $C_2H_5$-CH($CH_3$)O | CH($CH_3$)-$CH_2$ | O-(2-pyridyl) |
| 2 | $C_2H_5$-CH($CH_3$)O | $CH_2$-CH($CH_3$) | O-(2-pyridyl) |
| 3 | $C_2H_5$-CH($CH_3$)O | $CH_2$-$CH_2$ | O-(2-pyridyl) |
| 4 | $(CH_3)_2$C=CH-$CH_2$O | $CH_2$-$CH_2$ | O-(2-pyridyl) |
| 5 | $(CH_3)_2$C=CH-$CH_2$O | $CH_2$-CH($CH_3$) | O-(2-pyridyl) |
| 6 | $(CH_3)_2$C=CH-$CH_2$O | CH($CH_3$)-$CH_2$ | O-(2-pyridyl) |
| 7 | $C_2H_5$-CH($CH_3$)O | $CH_2$-$CH_2$ | O-(6-F-2-pyridyl) |
| 8 | $C_2H_5$-CH($CH_3$)O | $CH_2$-$CH_2$ | O-(6-Cl-2-pyridyl) |
| 9 | $C_2H_5$-CH($CH_3$)O | $CH_2$-CH($CH_3$) | O-(6-F-2-pyridyl) |
| 10 | $C_2H_5$-CH($CH_3$)O | CH($CH_3$)-$CH_2$ | O-(6-F-2-pyridyl) |
| 11 | $(CH_3)_2$C=CH-$CH_2$O | $CH_2$-$CH_2$ | O-(6-F-2-pyridyl) |
| 12 | $C_2H_5$-CH($CH_3$)O | $CH_2CH_2$ | O-(2-pyrimidinyl) |
| 13 | $C_2H_5$-CH($CH_3$)O | $CH_2$-CH($CH_3$) | O-(2-pyrimidinyl) |
| 14 | $C_2H_5$-CH($CH_3$)O | CH($CH_3$)-$CH_2$ | O-(2-pyrimidinyl) |
| 15 | $C_2H_5$-CH($CH_3$)S | $CH_2CH_2$ | O-(2-pyrimidinyl) |
| 16 | $C_2H_5$-CH($CH_3$)S | $CH_2$-CH($CH_3$) | O-(2-pyrimidinyl) |
| 17 | $(CH_3)_2$C=CH-$CH_2$O | $CH_2CH_2$ | O-(2-pyrimidinyl) |
| 18 | $(CH_3)_2$CH-O | $CH_2CH_2$ | O-(2-pyrimidinyl) |
| 19 | $(C_2H_5)_2$CH-O | $CH_2$-$CH_2$ | O-(2-pyrimidinyl) |
| 20 | $C_2H_5$-CH($CH_3$)-O | $CH_2$-$CH_2$ | S-(2-pyrimidinyl) |
| 21 | $C_2H_5$-CH($CH_3$)-S | $CH_2$-$CH_2$ | S-(2-pyrimidinyl) |
| 22 | $C_2H_5$-CH($CH_3$)-O | $CH_2$-CH($CH_3$) | S-(2-pyrimidinyl) |
| 23 | $(CH_3)_2$C=CH-$CH_2$O | $CH_2$-$CH_2$ | S-(2-pyrimidinyl) |
| 24 | n$C_3H_7$-CH($CH_3$)-O | $CH_2CH_2$ | S-(2-pyrimidinyl) |
| 25 | n$C_4H_9$-CH($CH_3$)-O | $CH_2CH_2$ | S-(2-pyrimidinyl) |
| 26 | $(CH_3)_2$CH-$CH_2$CH($CH_3$)O | $CH_2CH_2$ | S-(2-pyrimidinyl) |
| 27 | $CH_3CH_2$CH($CH_3$)O | $CH_2CH_2$ | O-(1,3-thiazol-2-yl) |

Continued..

TABLE I : Compounds of formula (A) wherein $(W)_{m'}$ is 0

| Cpd. | R-W[1] | $CH(R_1)-(CH_2)_n-CHR_2$ | X-$R_3$ |
|---|---|---|---|
| 28 | $CH_3CH_2CH(CH_3)O$ | $CH_2-CH(CH_3$ | O-(1,3-thiazol-2-yl) |
| 29 | $CH_3CH_2CH(CH_3)O$ | $CH(CH_3)-CH_2$ — | O-(1,3-thiazol-2-yl) |
| 30 | $CH_3CH_2CH(CH_3)S$ | $CH_2-CH_2$ | O-(1,3-thiazol-2-yl) |
| 31 | $(CH_3)_2C=CHCH_2O$ | $CH_2-CH_2$ | O-(1,3-thiazol-2-yl) |
| 32 | $CH_3CH_2CH(CH_3)O$ | $CH_2-CH_2$ | O-(2-thiazolin-2-yl) |
| 33 | $CH_3CH_2CH(CH_3)O$ | $CH_2CH(CH_3)$ | O-(2-thiazolin-2-yl) |
| 34 | $CH_3CH_2CH(CH_3)O$ | $CH_2CH_2$ | S-(2-thiazolin-2-yl) |
| 35 | $CH_3CH_2CH(CH_3)S$ | $CH_2CH_2$ | S-(2-thiazolin-2-yl) |
| 36 | $CH_3CH_2CH(CH_3)O$ | $CH_2-CH(CH_3)$ | S-(2-thiazolin-2-yl) |
| 37 | $(CH_3)_2C=CH(CH_3)O$ | $CH_2CH_2$ | S-(2-thiazolin-2-yl) |
| 38 | $(CH_3)_2CH-O$ | $CH_2-CH_2$ | S-(2-thiazolin-2-yl) |
| 39 | $CH_3CH_2CH(CH_3)O$ | $CH_2-CH(CH_3)$ | S-(1,3-thiazol-2-yl) |
| 40 | $CH_3CH_2CH(CH_3)O$ | $CH_2CH_2$ | S-(2-pyridyl) |
| 41 | $CH_3CH_2CH(CH_3)S$ | $CH_2CH_2$ | S-(2-pyridyl) |
| 42 | $CH_3CH_2CH(CH_3)O$ | $CH_2CH(CH_3)$ | S-(2-pyridyl) |
| 43 | $(CH_3)_2C=CH(CH_3)O$ | $CH_2CH_2$ | S-(2-pyridyl) |
| 44 | $CH_3CH_2CH(CH_3)O$ | $CH_2-CHCl$ | S-(2-pyrimidinyl) |
| 45 | $CH_3(CH_2)_2CH(CH_3)O$ | $CH_2CH_2$ | O-(2-pyridyl) |
| 46 | $CH_3(CH_2)_3CH(CH_3)O$ | $CH_2CH_2$ | O-(2-pyridyl) |
| 47 | $(C_2H_5)_2CH-O$ | $CH_2CH_2$ | O-(2-pyridyl) |
| 48 | $(CH_3)_2CH-O$ | $CH_2CH_2$ | O-(2-pyridyl) |
| 49 | $CH_3CH_2CH(CH_3)S$ | $CH_2CH(CH_3)$ | O-(1,3-thiazol-2-yl) |
| 50 | $CH_3(CH_2)_2CH(CH_3)O$ | $CH_2CH_2$ | O-(1,3-thiazol-2-yl) |
| 51 | $CH_3(CH_2)_3CH(CH_3)O$ | $CH_2CH_2$ | O-(1,3-thiazol-2-yl) |
| 52 | $(CH_3)_2CH-CH_2CH(CH_3)O$ | $CH_2CH_2$ | O-(1,3-thiazol-2-yl) |
| 53 | $(CH_3)_2CH-O$ | $CH_2CH_2$ | O-(1,3-thiazol-2-yl) |
| 54 | $(C_2H_5)_2CH-O$ | $CH_2CH_2$ | O-(1,3-thiazol-2-yl) |
| 55 | $CH_3CH_2CH(CH_3)O$ | $CH_2CH_2$ | S-(1,3-thiazol-2-yl) |
| 56 | $CH_3CH_2CH(CH_3)S$ | $CH_2CH(CH_3)$ | S-(2-thiazolin-2-yl) |

Continued... -11- 133-0632

TABLE I : Compounds of formula (A) wherein $(W)_{m'}$ is 0

| Cpd. | R-W[1] | $CH(R_1)-CH_2)_n-CHR_2$ | | X-$R_3$ |
|---|---|---|---|---|
| 57 | $CH_3(CH_2)_2CH(CH_3)O$ | $CH_2CH_2$ | | S-(2-thiazolin-2-yl) |
| 58 | $CH_3(CH_2)_3CH(CH_3)O$ | $CH_2CH_2$ | | S-(2-thiazolin-2-yl) |
| 59 | $(CH_3)_2CH-CH_2CH(CH_3)O$ | $CH_2CH_2$ | — | S-(2-thiazolin-2-yl) |
| 60 | $(C_2H_5)_2CH-O$ | $CH_2CH_2$ | | S-(2-thiazolin-2-yl) |
| 61 | $(CH_3)_2CH-O$ | $CH_2CH_2$ | | S-(2-thiazolin-2-yl) |

nmr (($CDCl_3$) characterisation of compounds of Table I:

Cpd. 1 : δ 0.8-1.8 (m, 11H), 3.9-4.8 (m, 4H), 6.6-7.0 (m, 6H), 7.3-7.7 (m, 1H) and 8.1-8.3 ppm (m, 1H).

**Cpd. 2** : δ 0.8-1.8 (m, 11H), 3.9-4.3 (m, 3H), 5.4-5.8 (m, 1H), 6.7-7.0 (m, 6H, 7.3-7.7 (m, 1H) and 8.1-8.3 ppm (2d, 1H).

**Cpd. 3** : δ 0.8-1.9 (m, 8H), 4.1-4.4 (m, 3H), 4.6-4.8 (m, 2H), 6.7-7.0 (m, 2H), 6.7-7.0 (m, 6H), 7.3-7.7 (m, 1H) and 8.1-8.3 ppm (m, 1H).

**Cpd. 4** : δ 1.8 (2s, 6H), 4.1-4.7 (m, 6H), 5.4 (t, 1H), 6.8-6.9 (m, 6H), 7.3-7.7 (m, 1H) and 8.0-8.2 ppm (m, 1H).

**Cpd. 5** : δ 1.6 (d, 3H), 1.9 (2s, 6H), 4.2-4.7 (m, 4H), 5.5 (t, 1H), 6.8-6.9 (m, 6H), 7.3-7.7 (m, 1H) and 8.0-8.2 ppm (m, 1H).

**Cpd. 7** : δ 0.8-1.9 (m,8H), 3.9-4.4 (m,3H), 4.5-4.7 (m,2H), 6.3-6.7 (m,2H), 6.8 (s, 4H) and 7.3-7.8 ppm (q, 1H).

**Cpd. 12** : δ 0.95 (m, 3H), 1.23 (d, 3H, J=6Hz), centered at 4.18 and 4.23(m, 5H) and 8.53 ppm (d, 2H, J=5Hz).

**Cpd. 13** : δ 0.96 (m, 3H), 1.25 (d, 3H, J=6Hz), 1.50 (d, 3H, J=6Hz), 5.57 (sextet, 1H, J=6Hz) and 8.54 ppm (d, 2H, J=5Hz).

**Cpd. 15** : δ 0.8-1.1 (t, 3H), 1.2 (d, 3H), 1.2-1.7 (m, 2H), 1.7-2.2 (h, 1H), 4.2-4.9 (m,4H), 6.9 (d,2H), 6.8-7.1 (m,1H), 7.4 (d,2H) and 8.5 ppm (d,2H)-

**Cpd. 16** : δ 0,8-1.1 (t, 3H), 1.2 (d, 3H), 1.3-1.9 (m, 2H), 2.7-3.3 (h, 1H), 3.9-4.6 (m, 2H), 5.4-5.7 (h, 1H), 6.8 (d, 2H)), 6.7-7.1 (m, 1H), 7.3 (d, 2H) and 8.5 ppm (d, 2H).

**Cpd. 17** : δ 1.6-1.8 (2ds, 6H), 4.2-4.8 (m,6H), 5.3-5.6 (t,1H), 6.9 (s,4H), 6.8-7.0 (m, 1H) and 8.5 ppm (d, 2H).

**Cpd. 18** : δ 1.2 (d, 6H), 4.2-4.8 (m,5H), 6.8 (s, 4H), 6.8-7.0 (m, 1H) and 8.4 ppm (d, 2H).

**Cpd. 19** : δ 0.9 (t, 6H), 1.2-1.9 (m, 4H), 4.0 (p. 1H), 4.2-4.4 (m, 2H), 4.6-4.9 (m, 2H), 6.9 (s, 4H), 6.8-7.0 (m, 1H) and 8.5 (d, 2H).

**Cpd. 20** : δ 0.95 (m, 3H), 1.23 (d, 3H, J=6Hz), 3.52 (m, 2H) and 8.53 ppm (d, 2H, J=5Hz).

**Cpd. 21** : δ 0.8-1.1 (t, 3H), 1.2 (d, 3H), 1.2-1.8 (m, 2H), 2.7-3.3 (h, 6H), 3.4-3.7 (t, 2H), 4.2-4.4 (t, 2H), 6.8-7.0 (m, 1H), 7.4 (d, 2H) and 8.5 ppm (d, 2H).

**Cpd. 23** : δ 0.94 (m, 3H), 1.24 (d, 3H, J=6Hz), 3.50 (t, 2H, J=7Hz), 4.22 (t, 2H, J=7Hz), 6.85 (s, 4H) and 8.49 ppm (d, 2H, J=5Hz).

**Cpd. 24** : δ 0.90 (m, 3H), 1.25 (d, 3H, J=6Hz), 3.51 (m, 2H), centered at 4.19 (m, 3H), 6.84 (m, 4H) and 8.51 ppm (d, 2H, J=5Hz).

**Cpd. 25** : δ 0.92 (m, 6H), 1.23 (d, 3H, J=6Hz), 3.50 (t, 2H, J=7Hz), 4.21 (m, 2H), 6.84 (s, 4H) and 8.49 ppm (d, 2H, J=5Hz).

**Cpd. 27** : δ 0.95 (t, 3H, J=7.25Hz), 1.24 (d, 3H, J=5.9Hz), 1.62 (m, 2H), 4.12 (m, 3H), 4.72 (m, 2H), 6.65 (d, 1H, J=2.2Hz) and 7.09 ppm (d, 1H, J=3.7Hz).

**Cpd. 28** : δ 0.95 (t, 3H), 1.23 (d, 3H, J=5.9Hz), 1.60 (m, 5H), 4.11 (m, 3H), 5.44 (m, 1H), 6.62 (d, 1H, J=3.7Hz), 6.82 (s, 4H) and 7.09 ppm (d, 1H, J=3.9Hz).

**Cpd. 30** : δ 0.98 (m, 3H), 1.21 (d, 3H, J=7Hz), centered at 4.29 and 4.75 (m, 4H), 6.67 (d, 1H, J=4Hz), 6.86 (m, 2H), 7.11 (d, 1H, J=4Hz) and 7.37 ppm (m, 2H).

**Cpd. 31** : δ 1.72 (d, 6H, J=5.5Hz), 4.20 (m, 2H), 4.41 (d, 2H, J=6.6Hz), 4.69 (m, 2H), 6.62 (m, 1H), 6.82 (s, 4H) and 7.08 ppm (m, 1H).

**Cpd. 34** : δ 0.96 (t, 3H, J=7.5Hz), 1.25 (d, 3H, J=6.2Hz), 1.66 (m, 2H), 3.44 (m, 4H), 4.21 (m, 5H) and 6.82

ppm (s, 4H).
**Cpd. 35** : δ 0.98 (m, 3H), 1.20 (d, 3H, J=7Hz), 3.42 (m, 4H), 4.21 (m, 4H), 6.85 (m, 2H) and 7.36 ppm (m, 2H).
**Cpd. 38** : δ 1.2 (d, 6H), 3.2-3.6 (m, 4H), 4.1-4.6 (m, 5H) and 6.8 ppm (s, 4H).
**Cpd. 40** : δ 0.8-1.1 (t, 3H), 1.1-1.3 (d, 3H), 1.3-1.9 (m, 2H), 3.4-3.7 (t, 2H), 4.0-4.3 (m, 3H), 6.9 (s, 4H), 6.7-7.6 (m, 3H) and 8.4 ppm (d, 1H).
**Cpd. 44** : δ 0.97 (m, 3H), 1.25 (d, 3H, J=6Hz), 4.22 (m, 1H, J=6Hz), 4.46 (d, 2H, J=6.Hz), 6.38 (t, 1H, J=6Hz) and 8.64 ppm (d, 2H, J=5Hz).
**Cpd. 45** : δ 0.94 (m, 3H), 1.24 (d, 3H, J=6Hz), 4.25 (m, 2H), 4.65 (m, 2H), 6.85 (s, 4H), 7.54 (m, 1H) and 8.13 ppm (m, 1H).
**Cpd. 46** : δ 0.90 (m, 3H), 1.24 (d, 3H, J=6Hz), 4.25 (m, 2H), 4.65 (m, 2H), 6.84 (s, 4H), 7.54 (m, 1H) and 8.13 ppm (m, 1H).
**Cpd. 47** : δ 0.95 (t, 6H), 1.2-2.0 (m, 4H), 3.8-4.2 (p, 1H), 4.2-4.4 (t, 2H), 4.5-4.8 (t, 2H), 6.7-7.0 (m, 2H), 6.8 (s, 4H), 7.3-7.7 (m, 1H) and 8.0-8.3 ppm (m, 1H).
**Cpd. 48** : δ 1.2 (d, 6H), 4.1-4.8 (m, 5H), 6.8 (s, 4H), 6.7-7.8 (m, 3H) and 8.0-8.5 ppm (m, 1H).
**Cpd. 49** : δ 0.98 (m, 3H), 1.2 (d, 3H, J=7Hz), 1.52 (d, 3H, J=7Hz), 2.95 (m, 1H), 4.14 (m, 2H), 5.43 (m, 1H), 6.64 (d, 1H, J=4Hz), 6.84 (m, 2H), 7.11 (d, 1H, J=4Hz) and 7.36 ppm (m, 2H).
**Cpd. 50** : δ 0.94 (m, 3H), 1.25 (d, 3H), J=6Hz), centered at 4.26 (m, 2H), centered at 4.74 (m, 2H), 6.68 (m, 1H), 6.83 (s, 4H) and 7.11 ppm (m, 1H).
**Cpd. 51** : δ 0.90 (m, 3H), 1.25 (d, 3H, J=6Hz), 4.05-4.31 (m, 3H), 4.73 (m, 2H), 6.67 (d, 1H, J=4Hz), 6.83 (s, 4H) and 7.11 ppm (d, 1H, J=4Hz).
**Cpd. 52** : δ 0.92 (m, 6H), 1.21 (d, 3H, J=6Hz), centered at 4.25 (m, 2H), centered at 4.73 (m, 2H), 6.68 (m, 1H), 6.75 (s, 4H) and 7.12 (m, 1H).
**Cpd. 53** : δ 1.2 (d, 6H), 4.1-4.8 (m, 5H), 6.6 (d, 1H), 6.8 (s, 4H) and 7.1 ppm (d, 1H).
**Cpd. 54** : δ 0.95 (t, 6H), 1.201.9 (m, 4H), 3.8-4.2 (m, 1H), 4.1-4.4 (m, 2H), 4.6-4.8 (m, 2H), 6.6 (d, 1H), 6.8 (s, 4H) and 7.1 (d, 1H).
**Cpd. 55** : δ 0.96 (m, 3H), 1.24 (d, 3H, J=6Hz), 3.57 (m, 2H), 4.07-4.30 (m, 3H), 6.80 (s, 4H), 4.19 (m, 1H) and 7.65 (m, 1H).
**Cpd. 56** : δ 0.97 (m, 3H), 1.19 (d, 3H, J=7Hz), 1.46 (d, 3H, J=7Hz), 2.94 (m, 1H), 3.32 (t, 3H, J=8Hz), 3.92-4.29 (m, 4H), 6.88 (m, 2H) and 7.35 ppm (m, 2H).
**Cpd. 57** : δ 0.94 (m, 3H), 1.25 (d, 3H, J=6Hz), 3.30-3.51 (m, 4H), 4.11-4.30 (m, 4H) and 6.82 ppm (s, 4H).
**Cpd. 58** : δ 0.90 (m, 3H), 1.24 (d, 3H, J=6Hz), centered at 3.39 (m, 4H), centered at 4.19 (m, 5H) and 6.81 ppm (s, 4H).
**Cpd. 59** : δ 0.92 (m, 6H), 1.23 (d, 3H, J=6Hz), centered at 3.40 (m, 4H), 3.99-4.40 (m, 5H) and 6.82 ppm (s, 4H); and
**Cpd. 60** : δ 0.95 (tt, 6H), 1.2-1.9 (m, 4H), 3.4 (q, 4H), 3.8-4.3 (m, 5H) and 6.9 ppm (s, 4H).

## EXAMPLE 7

The compounds (a) 2-{2-[4-(1-methylpropoxy)phenoxy] -ethoxy}pyridine (compound 3), (b) 2-{1-methyl-2-[4-(1-methoypropoxy) -phenoxy]ethoxy}-pyridine (compound 2), (c) 6-fluoro-2-{2-[4-1-methylpropoxy)phenoxy]ethoxy}pyridine (compound 7), and (d) 2-2{2-[4-(1-methylpropoxy) phenoxy]-ethoxy}pyrimidine (compound 12), are tested for contact activity on houseflies by the following method.

Third instar, post-feeding wandering Musca domestica L. larvae are individually treated topically with 1 µl of the test compound in acetone at different dose rates. Additional larvae are treated identically with 1 µl of acetone as the control. Larvae are held in covered containers for 7 days at 31° and 16 hour photoperiod. The assay effect is expressed as $ED_{50}$, which is the dose, in ug per larva, required to cause an effect in 50% of the test insects. Effects observed include direct toxicity (larval death); delayed toxicity (pupal death); and juvenile hormone activity, such as failure of adults to emerge completely, chitin inhibition, distortion of cuticle and pupation abnormalities. Each of the above tested compounds had an $ED_{50}$ of less than 0.0050 µg/larva.

## EXAMPLE 8

The compounds (a) 2-{2-[4-(1-methylpropoxy)phenoxy]ethoxy}pyridine (compound 3), and (b) 2-{1-methyl-2-[4-(1-methylpropoxy)phenoxy]ethoxy} pyridine (compound 2) are tested for activity on the yellow fever mosquito as follows.

Late fourth instar Aedes aegypti larvae (generally 5 days post-hatching) are placed in plastic containers

with 50 ml tap water into which has been mixed 50 µl of acetone dilution of the test compound at the concentration to be tested. A few drops of a liver powder suspension are added as a food source. The containers are covered and held at 28°, 16 hour photoperiod until all larvae or pupae are either dead or have emerged as adults. The assay effect is expressed as $EC_{50}$, which is the concentration, in ppm required to cause an effect in 50% of the test insects. Effects observed include direct toxicity (larval death) and juvenile hormone activity such as pupal mortality and failure of adults to emerge completely. Each of the above tested compounds had an $EC_{50}$ of less than 0.0050 ppm.

## Claims

### Claims for the following Contracting States : BE, CH, DE, FR, GB, IT, LI, LU, NL, SE

**1.** Compounds of formula (A)

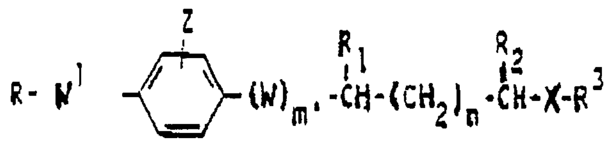

(A)

wherein m' is zero or one;
n is zero, one or two;
W is oxygen, sulfur, $NR^4$ or carbony];
$W^1$ is oxygen, sulfur, $NR^4$, carbonyl, sulfinyl or. sulfonyl;
X is oxygen or sulfur;
Z is hydrogen, $C_{1-8}$ alkyl, $C_{1-8}$ haloalkyl or halogen;
R is $C_{3-8}$alkyl, $C_{2-8}$alkenyl, $C_{2-8}$alkynyl, $C_{1-8}$haloalkyl, $C_{2-8}$haloalkenyl, $C_{2-8}$haloalkyny, $C_{2-10}$alkylthioalkyl $C_{3-8}$cycloalkyl, $C_{3-8}$halocycloalkyl, $C_{4-12}$cycloalkylalkyl or heterocycloalkyl;
each of $R^1$ and $R^4$ is independently hydrogen or $C_{1-8}$alkyl;
$R^2$ is hydrogen, $C_{1-8}$alkyl or halogen; and
either $R^3$ is an aromatic nitrogen-containing heteroring selected from pyridyl, 3-pyridazinyl, 2-pyrimidinyl, pyrazinyl, triazinyl or 2-thiazolyl which aromatic nitrogen-containing heteroring may be unsubstituted or substituted by one or more substituents selected from halogen, $C_{1-8}$alkyl, $C_{1-8}$haloalkyl, $C_{1-8}$alkoxy, $C_{1-8}$alkylthio and $NO_2$,
or is a group ($G_1$):

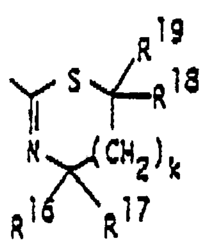

($G_1$)

in which k is zero or one; and
each of $R^{16}$, $R^{17}$, $R^{18}$ and $R^{19}$ is independently hydrogen or methyl,
provided that

a) when

m' is one,
X, $W^1$ and W are oxygen,
$R^3$ is 2-pyridyl,
n is 0,
and Z, $R^1$ and $R^2$ are H,

then R is not 2-methylpropyl, isopentyl, n-butyl, n-propyl, n-pentyl, 2-methylbutyl, 3,3-dimethylbutyl,

2,2-dimethylpropyl, 2-methyl-2-propenyl or 2-propenyl;

or b) when
R is isopentyl
m' is one,
$W^1$ and W are oxygen,
n is 0,
X is oxygen,
and Z, $R^1$ and $R^2$ are H,
then $R^3$ is not 2-pyrimidinyl or 2-thiazolyl;

or c) when

R is isopentyl
m' is one,
$W^1$ and W are oxygen,
n is 0
X is oxygen,
Z and $R^1$ are H,
and $R^2$ is methyl,
then $R^3$ is not 2-pyridyl, 2-pyrimidinyl or 2-thiazolyl;

or d) when

R is isopentyl
m' is one,
$W^1$ and W are oxygen,
n is 0,
X is oxygen,
Z and $R^2$ are H,
and $R^1$ is methyl

then $R^3$ is not 2-pyrimidinyl, 2-pyridyl or 2-thiazolyl;

or e) when

R is isopentyl
m' is one,
$W^1$ and W are oxygen,
n is 0,
X is sulfur,
and Z, $R^1$ and $R^2$ are H,

then $R^3$ is not 2-thiazolin-2-yl, 2-pyridyl or 2-pyrimidinyl;

or f) when

R is isopentyl
m' is one,
$W^1$ and W are oxygen,
n is 0,
X is oxygen,
Z is Cl in ortho position of $RW^1$,

and $R^1$ and $R^2$ are H,

then $R^3$ is not 2-pyridyl or 2-thiazolyl;

or g) when

m' is one,
X, $W^1$ and W are oxygen,
$R^3$ is 2-thiazolyl,
n is 0,
and Z, $R^1$ and $R^2$ are H,

then R is not isobutyl.

2. A compound of Claim 1, wherein R is $C_{3-6}$alkyl or $C_{3-6}$alkenyl.

3. A compound of Claim 1 or 2, wherein $R^3$ is a N-containing heteroring selected from 2-pyridyl, 2-pyrimidinyl , 1,3-thiazol-2-yl, which neteroring may be unsubstituted or monosubstituted by halogen, or is 2-thiazolin-2-yl.

4. A compound of Claim 3, wherein m' is one and n is zero.

5. A compound of Claim 4, wherein Z is hydrogen, W is oxygen, $W^1$ is oxygen or sulphur, $R^1$ is hydrogen or methyl and $R^2$ is hydrogen, methyl or halogen.

6. A pest controlling composition comprising a compound of any one of Claims 1 to 5 and a diluent.

7. A method of controlling pests which comprises applying to the pest or locus a pest controlling amount of a compound of any one of Claims 1 to 5.

8. Process of preparing compounds of formula (A), stated in Claims 1 to 5 which comprises etherifying a compound of formula (I)

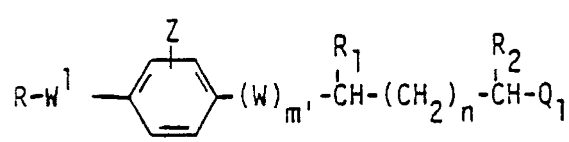

(I)

wherein R, $R^1$, $R^2$, $W^1$, W, Z, m' and n are as defined in Claim 1,
and $Q_1$ is OH, SH or a leaving group capable of being split off under the reaction conditions
with a compound of formula (II)

$Q_2$ - $R^3$ (II)

wherein $R^3$ is as defined in Claim 1,
and $Q_2$ is a leaving group capable of being split off under the reaction conditions where $Q_1$ is OH or SH, or is OH or SH where $Q_1$ is a leaving group,
followed, where desired, by halogenation of the thus obtained compounds of formula (A), wherein $R^2$ is H, X is S and W and $W^1$ are 0 to compounds of formula (A) wherein $R^2$ is halogen, X is S and W and $W^1$ are 0.

9. A compound of Claim 1, wherein R is 2-butyl or 3-methyl-2-butenyl.

10. A compound of Claim 1, wherein R-$W^1$ is $C_2H_5$-CH($CH_3$)O, $(W)_{m'}$ is 0, X-$R_3$ is 0-(2-pyridyl), and CH-($R_1$)-$(CH_2)_n$-$CHR_2$ is either $CH_2$-CH($CH_3$) or $CH_2CH_2$.

**Claims for the following Contracting State : AT**

1. A pest controlling composition comprising a compound of formula (A)

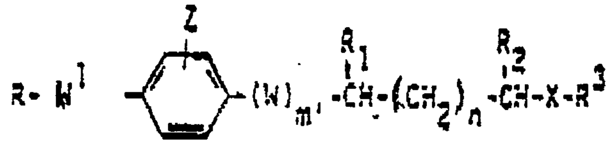

(A)

wherein M' is zero or one;
n is zero, one or two;
W is oxygen, sulfur, $NR^4$ or carbony];
$W^1$ is oxygen, sulfur, $NR^4$, carbonyl, sulfinyl or sulfonyl;
X is oxygen or sulfur;
Z is hydrogen, $C_{1-8}$ alkyl, $C_{1-8}$ haloalkyl or halogen;
R is $C_{3-8}$alkyl, $C_{2-8}$alkenyl, $C_{2-8}$alkynyl, $C_{1-8}$haloalkyl, $C_{2-8}$haloalkenyl, $C_{2-8}$haloalkyny], $C_{2-10}$alkylthioalkyl $C_{3-8}$cycloalkyl, $C_{3-8}$halocycloalkyl, $C_{4-12}$cycloalkylalkyl or heterocycloalkyl;
each of $R^1$ and $R^4$ is independently hydrogen or $C_{1-8}$alkyl;
$R^2$ is hydrogen, $C_{1-8}$alkyl or halogen; and
either $R^3$ is an aromatic nitrogen-containing heteroring selected from pyridyl, 3-pyridazinyl, 2-pyrimidinyl, pyrazinyl, triazinyl or 2-thiazolyl which aromatic nitrogen-containing heteroring may be unsubstituted or substituted by one or more substituents selected from halogen, $C_{1-8}$alkyl, $C_{1-6}$haloalkyl, $C_{1-8}$alkoxy, $C_{1-8}$alkylthio and $NO_2$,
or is a group ($G_1$):

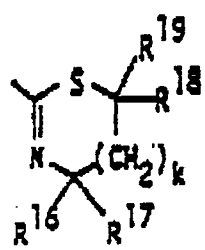

($G_1$)

in which k is zero or one; and
each of $R^{16}$, $R^{17}$, $R^{18}$and $R^{19}$is independently hydrogen or methyl, in association with a diluent provided that

a) when

m' is one,
X, $W^1$ and W are oxygen,
$R^3$ is 2-pyridyl,
n is 0,
and Z, $R^1$ and $R^2$ are H,

then R is not 2-methylpropyl, isopentyl, ethyl, n-butyl, n-propyl, n-pentyl, 2-methylbutyl, 3,3-dimethylbutyl, 2,2-dimethylpropyl, 2-methyl-2-propenyl or 2-propenyl:

or b) when

R is isopentyl
m' in one,
$W^1$ and W are oxygen,
n is 0,

X is oxygen,
and Z, $R^1$ and $R^2$ are H,
then $R^3$ is not 2-pyrimidinyl or 2-thiazolyl;

or c) when

R is isopantyl
m' is one,
$W^1$ and W are oxygen,
n is 0
X is oxygen,
Z and $R^1$ are H,
and $R^2$ is methyl,
then $R^2$ is not 2-pyridyl, 2-pyrimidinyl or 2-thiazolyl;

or d) when

R is isopentyl
m' is one,
$W^1$ and W are oxygen,
n is 0,
X is oxygen,
Z and $R^3$ are H,
and $R^1$ is methyl

then $R^3$ is not 2-pyrimidinyl, 2pyridyl or 2-thiazolyl;

or e) when

R is isopentyl
m' is one,
$W^1$ and W are oxygen,
n is 0,
X is sulfur,
and Z, $R^1$ and $R^2$ are H,

then $R^3$ is not 2-thiazolin-2-yl, 2pyridyl or 2-pyrimidinyl;

or f) when

R is isopentyl
m' is one,
$W^1$ and W are oxygen,
n is 0,
X is oxygen,
Z is Cl in ortho position of $RW^1$,
and $R^1$ and $R^2$ are H,

then $R^3$ is not 2-pyridyl or 2-thiazolyl;

or g) when

m' is one,
X, $W^1$ and W are oxygen,
$R^3$ is 2-thiazolyl,
n is 0,
and Z, $R^1$ and $R^2$ are H,
8

then R is not isobutyl.

2. A composition of Claim 1, wherein R is $C_{3-6}$alkyl or $C_{3-6}$alkenyl.

3. A composition of Claim 1 or 2, wherein $R^3$ is a N-containing heteroring selected from 2-pyridyl, 2-pyrimidinyl, 1,3-thiazol-2-yl, which heteroring may be unsubstituted or monosubstituted by halogen, or is 2-thiazolin-2-yl.

4. A composition of Claim 3, wherein m' is one and n is zero.

5. A composition of Claim 4, wherein Z is hydrogen, W is oxygen, $W^1$ is oxygen or sulphur, $R^1$ is hydrogen or methyl and $R^2$ is hydrogen, methyl or halogen.

6. A method of controlling pests which comprises applying to the pest or locus a pest controlling amount of a compound of any one of Claims 1 to 5.

7. Process of preparing compounds of formula (A), stated in Claims 1 to 5 which comprises etherifying a compound of formula (I)

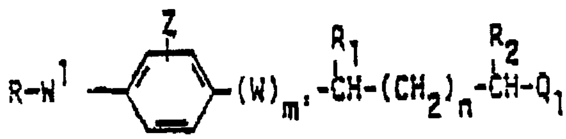

(I)

wherein R, $R^1$, $R^2$, $W^1$, W, Z, m' and n are as defined in Claim 1,
and $Q_1$, is OH, SH or a leaving group capable of being split off under the reaction conditions
with a compound of formula (II)

$Q_2$ - $R^3$ (II)

wherein $R^3$ is as defined in Claim 1,
and $Q_2$ is a leaving group capable of being split off under the reaction conditions where $Q_1$ is OH or SH, or is OH or SH where $Q_1$ is a leaving group,
followed, where desired, by haloganation of the thus obtained compounds of formula (A), wherein $R^2$ is H, X is S and W and $W^1$ are O to compounds of formula (A) wherein $R^2$ is halogen, X is S and W and $W^1$ are O.

8. A composition of Claim 1, wherein R is 2-butyl or 3-methyl-2- butenyl.

9. A composition of Claim 1, wherein R-$W^1$ is $C_2H_5$-$CH(CH_3)O$, $(W)_{m'}$ is O, x-$R_3$ is O-(2-pyridyl), and CH-$(R_1)$-$(CH_2)_n$-$CHR_2$ is either $CH_2$-$CH(CH_3)$ or $CH_2CH_2$.

**Revendications**
**Revendications pour les Etats contractants suivants : BE, CH, DE, FR, GB, IT, LI, LU, NL, SE**

1. Les composés de formule (A)

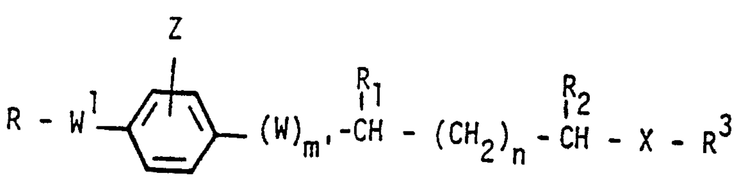

(A)

dans laquelle
m' signifie 0 ou 1,

n signifie 0, 1 ou 2,
W signifie l'oxygène, le soufre, $NR^4$ ou carbonyle,
$W^1$ signifie l'oxygène, le soufre, $NR^4$, carbonyle, sulfinyle ou sulfonyle,
X signifie l'oxygène ou le soufre,
Z signifie l'hydrogène, alkyle en $C_1$-$C_6$, haloalkyle en $C_1$-$C_8$ ou un halogène,
R signifie alkyle en $C_3$-$c_8$, alcényle en $C_2$-$C_6$, alcynyle en $C_2$-$C_8$ , haloalkyle en $c_1$-$c_8$, haloalcényle en $C_2$-$C_8$, haloalcynyle en $C_2$-$C_8$, alkylthioalkyle en $c_2$ -$C_{10}$, cycloalkyle en $C_3$-$C_8$,halocycloalkyle en $C_1$ -$C_8$, cycloalkyl-alkyle en $C_4$-$C_{12}$ ou hétérocycloalkyle,
chaque $R^1$ et $R^4$ signifie indépendamment l'hydrogène ou alkyle en $C_1$ -$C_8$,
$R^2$ signifie l'hydrogène, alkyle en $c_1$-$C_8$ ou un halogène et,
$R^3$ signifie un hérérocycle aromatique contenant de l'azote choisi parmi les groupes pyridyle, 3-pyridazinyle, 2-pyrimidinyle, pyrazinyle, triazinyle et 2-thiazolyle, lequel hétérocycle aromatique contenant de l'azote peut être non substitué ou substitué par un ou plusieurs substituants choisis parmi les halogènes et les groupes alkyle en $C_1$-$C_8$, haloalkyle en $C_1$-$C_8$, alcoxy en $C_1$-$C_8$, alkylthio en $C_1$-$C_8$ et $NO_2$, ou bien signifie un groupe ($G_1$):

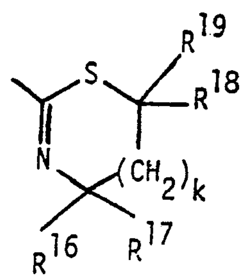

($G_1$)

où k signifie 0 ou 1 et
chaque $R^{16}$, $R^{17}$, $R^{18}$ et $R^{19}$ signifie indépendamment l'hydrogène ou méthyle,
avec les conditions que

a) R ne signifie pas un groupe 2-méthylpropyle, isopentyle, éthyle, n-butyle, n-propyle, n-pentyle, 2-méthylbutyle, 3,3-diméthylbutyle, 2,2-diméthylpropyle, 2-méthyl-2-propényle ou 2-propényle,

lorsque

m' signifie 1,
X, W' et W signifient l'oxygène,
$R^3$ signifie 2-pyridyle,
n signifie 0, et
Z R' et $R^2$ signifient H, ou

b) $R^3$ ne signifie pas un groupe 2-pyrimidinyle ou 2-thiazolyle

lorsque

R signifie isopentyle,
m' signifie 1,
$W^1$ et W signifient l'oxygène,
n signifie 0,
X signifie l'oxygène, et
Z, $R^1$ et $R^2$ signifient H ou

c) $R^3$ ne signifie pas un groupe 2-pyridyle, 2-pyrimidinyle ou 2-thiazolyle;

lorsque

R signifie isopentyle,
m' signifie 1,

$W^1$ et W signifient l'oxygène,
n signifie 0,
X signifie l'oxygène,
Z et $R^1$ signifient H et
$R^2$ signifie méthyle, ou

d) $R^3$ ne signifie pas un groupe 2-pyrimidinyle, 2-pyridyle ou 2-thiazolyle,

lorsque

R signifie isopentyle,
m' signifie 1,
$W^1$ et W signifient l'oxygène,
n signifie 0,
X signifie l'oxygène,
Z et $R^2$ signifient H, et
$R^1$ signifie méthyle, ou

e) $R^3$ ne signifie pas un groupe 2-thiazoline-2-yle, 2-pyridyle ou 2-pyrimidinyle,

lorsque

R signifie isopentyle,
m' signifie 1,
$W^1$ et W signifient l'oxygène,
n signifie 0,
X signifie le soufre, et
Z, $R^1$ et $R^2$ signifient H, ou

f) $R^3$ ne signifie pas un groupe 2-pyridyle ou 2-thiazolyle,

lorsque

R signifie isopentyle,
m' signifie 1,
$W^1$ et W signifient l'oxygène,
n signifie 0,
X signifie l'oxygène,
Z signifie Cl en position ortho de $RW^1$, et
$R^1$ et $R^2$ signifient H, ou

g) R ne signifie pas un groupe isobutyle,

lorsque

m' signifie 1,
X, $W^1$ et W signifient l'oxygène,
$R^3$ signifie 2-thiazolyle,
n signifie 0, et
Z, $R^1$ et $R^2$ signifient H.

**2.** Un composé selon la revendication 1, dans lequel R signifie alkyle en $C_3$-$c_6$ ou alcényle en $C_3$-$C_6$.

**3.** Un composé selon la revendication 1 ou 2, dans lequel $R^3$ signifie un hétérocycle contenant de l'azote choisi parmi les groupes 2-pyridyle, 2-pyrimidinyle, 1,3-thiazole-2-yle, lequel hétérocycle peut être non substitué ou monosubstitué par de l'halogène, ou signifie un groupe 2-thiazoline-2-yle.

**4.** Un composé selon la revendication 3, dans lequel m' signifie 1 et n signifie 0.

**5.** Un composé selon la revendication 4, dans lequel Z signifie l'hydrogène, W signifie l'oxygène, $W^1$ signifie l'oxygène ou le soufre, $R^1$ signifie l'hydrogène ou méthyle et $R^2$ signifie l'hydrogène, méthyle ou un halogène.

**6.** Une composition destinée à combattre les organismes nuisibles, qui comprend un composé selon l'une quelconque des revendications 1 à 5 et un diluant.

**7.** Une méthode de lutte contre les organismes nuisibles, qui comprend l'application sur l'organisme nuisible ou son habitat d'une quantité d'un composé selon l'une quelconque des revendications 1 à 5 combattant l'organisme nuisible.

**8.** Un procédé de préparation des composés de formule (A), spécifiés aux revendications 1 à 5, qui comprend l'estérification d'un composé de formule (I)

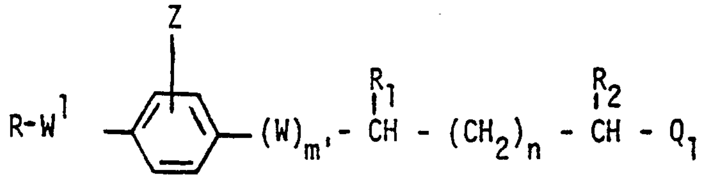

(I)

dans laquelle R, $R^1$, $R^2$, $W^1$, W, Z, m' et n sont tels que définis à la revendication 1, et $Q^1$ signifie 0H, SH ou un groupe éliminable capable d'être scindé sous les conditions de la réaction, avec un composé de formule (II)

$Q_2 - R^3$ (II)

dans laquelle $R^3$ est tel que défini à la revendication 1, et $Q_2$ signifie un groupe éliminable capable d'être scindé sous les conditions de la réaction lorsque $Q_1$ signifie OH ou SH, ou signifie OH ou SH lorsque $Q_1$ signifie un groupe éliminable, suivie, si on le désire, par l'halogénation des composés ainsi obtenus de formule (A) où $R^2$ signifie H, X signifie S et W et $W^1$ signifient 0, en composés de formule (A) où $R^2$ signifie un halogène, X signifie S et W et $W^1$ signifient 0.

**9.** Un composé selon la revendication 1, dans lequel R signifie un groupe 2-butyle ou 3-méthyl-2-butényle.

**10.** Un composé selon la revendication 1, dans lequel R-$W^1$ signifie $C_2H_5$-CH($CH_3$)O, $(W)_{m'}$ signifie 0, X-$R_3$ signifie 0-(2-pyridyle) et CH($R_1$ )-($CH_2$ )$_n$-$CHR_2$ signifie $CH_2$-CH($CH_3$) ou $CH_2CH_2$.

**Revendications pour l'Etat contractant suivant : AT**

**1.** Une composition destinée à combattre les organismes nuisibles qui comprend un composé de formule (A)

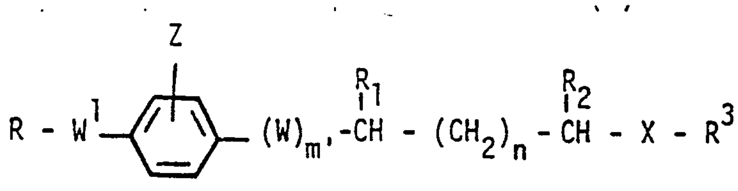

(A)

dans laquelle
m' signifie 0 ou 1,
n signifie 0, 1 ou 2,
W signifie l'oxygène, le soufre, $NR^4$ ou carbonyle,
$w^1$ signifie l'oxygène, le soufre, $NR^4$, carbonyle, sulfinyle ou sulfonyle,

X signifie l'oxygène ou le soufre,
Z signifie l'hydrogène, alkyle en $C_1$-$C_8$, haloalkyle en $C_1$-$C_8$ ou un halogène,
R signifie alkyle en $C_3$-$C_8$, alcényle en $C_2$-$C_8$, alcynyle en $C_2$-$C_8$, haloalkyle en $C_{1-8}$, haloalcényle en $C_2$-$C_8$, haloalcynyle en $C_2$-$C_8$, alkylthioalkyle en $C_2$-$C_{10}$, cycloalkyle en $C_3$-$C_8$,halocycloalkyle en $C_3$-$C_8$, cycloalkyl-alkyle en $C_4$-$C_{12}$ ou hétérocycloalkyle,
chaque $R^1$ et $R^4$ signifie indépendamment l'hydrogène ou alkyle en $C_1$-$C_8$,
$R^2$ signifie l'hydrogène, alkyle en $C_1$-$C_8$ ou un halogène et,
$R^3$ signifie un hérérocycle aromatique contenant de l'azote choisi parmi les groupes pyridyle, 3-pyridazinyle, 2-pyrimidinyle, pyrazinyle, triazinyle et 2-thiazolyle, lequel hétérocycle aromatique contenant de l'azote peut être non substitué ou substitué par un ou plusieurs substituants choisis parmi les halogènes et les groupes alkyle en $C_1$-$C_8$, haloalkyle en $C_1$-$C_8$, alcoxy en C1-C8, alkylthio en $C_1$-$C_8$ et $NO_2$, ou bien signifie un groupe ($G_1$):

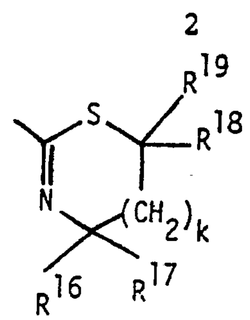

($G_1$)

où k signifie 0 ou 1 et
chaque $R^{16}$, $R^{17}$, $R^{18}$ et $R^{19}$ signifie indépendamment l'hydrogène ou méthyle,
en association avec un diluant,
avec les conditions que

a) R ne signifie pas un groupe 2-méthylpropyle, isopentyle, éthyle, n-butyle, n-propyle, n-pentyle, 2-méthylbutyle, 3,3-diméthylbutyle, 2,2-diméthylpropyle, 2-méthyl-2-propényle ou 2-propényle,

lorsque

m' signifie 1,
X, $W^1$ et W signifient l'oxygène,
$R^3$ signifie 2-pyridyle,
n signifie 0, et
Z $R^1$ et $R^2$ signifient H, ou

b) $R^3$ ne signifie pas un groupe 2-pyrimidinyle ou 2-thiazolyle

lorsque

R signifie isopentyle,
m' signifie 1,
$W^1$ et W signifient l'oxygène,
n signifie 0,
X signifie l'oxygène, et
Z, $R^1$ et $R^2$ signifient H, ou

c) $R^3$ ne signifie pas un groupe 2-pyridyle, 2-pyrimidinyle ou 2-thiazolyle;

lorsque

R signifie isopentyle,
m' signifie 1,

$W^1$ et W signifient l'oxygène,
n signifie 0,
X signifie l'oxygène,
Z et $R^1$ signifient H et
$R^2$ signifie méthyle, ou

d) $R^3$ ne signifie pas un groupe 2-pyrimidinyle, 2-pyridyle ou 2-thiazolyle,

lorsque

R signifie isopentyle,
m' signifie 1,
$W^1$ et W signifient l'oxygène,
n signifie 0,
X signifie l'oxygène,
Z et $R^2$ signifient H, et
$R^1$ signifie méthyle, ou

e) $R^3$ ne signifie pas un groupe 2-thiazoline-2-yle, 2-pyridyle ou 2-pyrimidinyle,

lorsque

R signifie isopentyle,
m' signifie 1,
$W^1$ et W signifient l'oxygène,
n signifie 0,
X signifie le soufre, et
Z, $R^1$ et $R^2$ signifient H, ou

f) $R^3$ ne signifie pas un groupe 2-pyridyle ou 2-thiazolyle,

lorsque

R signifie isopentyle,
m' signifie 1,
$W^1$ et W signifient l'oxygène,
n signifie 0,
X signifie l'oxygène,
Z signifie Cl en position ortho de $RW^1$, et
$R^1$ et $R^2$ signifient H, ou

g) R ne signifie pas un groupe isobutyle,

lorsque

m' signifie 1,
X, $W^1$ et W signifient l'oxygène,
$R^3$ signifie 2-thiazolyle,
n signifie 0, et
Z, $R^1$ et $R^2$ signifient H.

2. Une composition selon la revendication 1, dans laquelle R signifie alkyle en $C_3$-$C_6$ ou alcényle en $C_3$-$C_6$.

3. Une composition selon la revendication 1 ou 2, dans laquelle $R^3$ signifie un hétérocycle contenant de l'azote choisi parmi les groupes 2-pyridyle, 2-pyrimidinyle, 1,3-thiazole-2-yle, lequel hétérocycle peut être non substitué ou monosubstitué par de l'halogène, ou signifie un groupe 2-thiazoline-2-yle.

4. Une composition selon la revendication 3, dans laquelle m' signifie 1 et n signifie 0.

5. Une composition selon la revendication 4, dans laquelle Z signifie l'hydrogène, W signifie l'oxygène, $W^1$ signifie l'oxygène ou le soufre, $R^1$ signifie l'hydrogène ou méthyle et $R^2$ signifie l'hydrogène, méthyle ou un halogène.

6. Une méthode de lutte contre les organismes nuisibles, qui comprend l'application sur l'organisme nuisible ou son habitat d'une quantité d'un composé selon l'une quelconque des revendications 1 à 5 combattant l'organisme nuisible.

7. Un procédé de préparation des composés de formule (A), spécifiés aux revendications 1 à 5, qui comprend l'estérification d'un composé de formule (I)

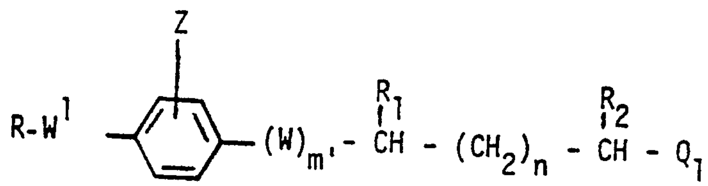

(I)

dans laquelle R, $R^1$, $R^2$, $W^1$, W, Z, m' et n sont tels que définis à la revendication 1, et $Q^1$ signifie OH, SH ou un groupe éliminable capable d'être scindé sous les conditions de la réaction, avec un composé de formule (II)

$Q_2$ - $R^3$ (II)

dans laquelle $R^3$ est tel que défini à la revendication 1, et $Q_2$ signifie un groupe éliminable capable d'être scindé sous les conditions de la réaction lorsque $Q_1$ signifie OH ou SH, ou signifie OH ou SH lorsque $Q_1$ signifie un groupe éliminable, suivie, si on le désire, par l'halogénation des composés ainsi obtenus de formule (A) où $R^2$ signifie H, X signifie S et W et $W^1$ signifient 0, en composés de formule (A) où $R^2$ signifie un halogène, X signifie S et W et $W^1$ signifient 0.

8. Une composition selon la revendication 1, dans laquelle R signifie un groupe 2-butyle ou 3-méthyl-2-butényle.

9. Une composition Selon la revendication 1, dans laquelle R-$W^1$ signifie $C_2H_5$-CH($CH_3$ )O, $(W)_{m'}$ signifie 0, X-$R_3$ signifie O-(2-pyridyle), et CH($R_1$ )-($CH_2$ $)_n$-$CHR_2$ signifie $CH_2$-CH($CH_3$) ou $CH_2CH_2$.

**Patentansprüche**

**Patentansprüche für folgende Vertragsstaaten : BE, CH, DE, FR, GB, IT, LI, LU, NL, SE**

1. Verbindungen der Formel (A)

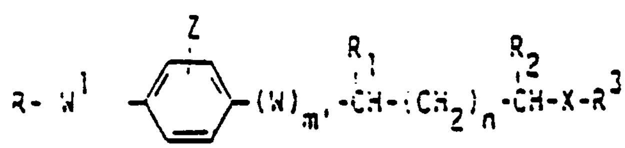

(A)

worin
m' 0 oder 1 ist;
n 0, 1 oder 2 ist;
W Sauerstoff, Schwefel, $NR^4$ oder ein Carbonylrest ist;
$W^1$ Sauerstoff, Schwefel, $NR^4$, eine Carbonyl-, Sulfinyl- oder Sulfonylgruppe ist;
X Sauerstoff oder Schwefel ist;

Z Wasserstoff, eine $C_1$-$C_8$-Alkyl-, $C_1$-$C_8$-Halogenalkylgruppe oder Halogen ist;
R eine $C_3$-$C_8$-Alkyl-, $C_2$-$C_8$-Alkenyl-, $C_2$-$C_8$-Alkinyl-, $C_1$-$C_8$-Halogenalkyl, $C_2$-$C_8$-Halogenalkenyl-, $C_2$-$C_8$-Halogenalkinyl-, $C_2$-$C_{10}$-Alkylthioalkyl-, $C_3$-$C_8$-Cycloalkyl-, $C_3$-$C_8$-Halogencycloalkyl-, $C_4$-$C_{12}$-Cycloalkylalkyl- oder Hetereocycloalkyl gruppe ist;
jeder der Reste $R^1$ und $R^4$ unabhängig Wasserstoff oder ein $C_1$-$C_8$-Alkylrest ist;
$R^2$ Wasserstoff, ein $C_1$-$C_8$-Alkylrest oder Halogen ist; und entweder
$R^3$ ein aromatischer Stickstoff enthaltender Heteroring ist, ausgewählt aus Pyridyl, 3-Pyridazinyl, 2-Pyrimidinyl, Pyrazinyl, Triazinyl oder 2-Thiazolyl, wobei der den Stickstoff enthaltende aromatische Heteroring unsubstituiert oder mit ein oder mehreren Substituenten, ausgewählt aus Halogen, einem $C_1$-$C_8$-Alkyl-, $C_1$-$C_8$-Halogenalkyl-, $C_1$-$C_8$-Alkoxy-, $C_1$-$C_8$-Alkylthiorest und $NO_2$, substituiert sein kann, oder eine Gruppe ($G_1$) ist:

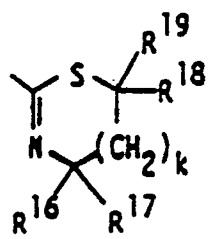

($G_1$)

worin k 0 oder 1 ist und jeder der Reste $R^{16}$, $R^{17}$, $R^{18}$ und $R^{19}$ unabhängig Wasserstoff oder ein Methylrest ist,
mit dem Vorbehalt, daß

a) wenn
m' 1 ist, X, $W^1$ und W Sauerstoff sind, $R^3$ ein 2-Pyridylrest ist,
n 0 ist, und Z, $R^1$ und $R^2$ H sind,
daß dann R kein 2-Methylpropyl-, Isopentyl-, Ethyl-, n-Butyl-, n-Propyl-, n-Pentyl-, 2-Methylbutyl-, 3,3-Dimethylbutyl-, 2,2-Dimethylpropyl-, 2-Methyl-2-propenyl- oder 2-Propenylrest ist;
oder b) wenn
R ein Isopentylrest ist, m' 1 ist, $W^1$ und W Sauerstoff sind, n 0 ist, X Sauerstoff ist, und Z, $R^1$ und $R^2$ H sind,
daß dann $R^3$ kein 2-Pyrimidinyl- oder 2-Thiazolylrest ist;
oder c) wenn
R ein Isopentylrest ist, m' 1 ist, $W^1$ und W Sauerstoff sind, n 0 ist, X Sauerstoff ist, Z und $R^1$ H sind, und $R^2$ eine Methylgruppe ist,
daß dann $R^3$ kein 2-Pyridyl-, 2-Pyrimidinyl- oder 2-Thiazolylrest ist;
oder d), wenn
R ein Isopentylrest ist, m' 1 ist, $W^1$ und W Sauerstoff sind, n 0 ist, X Sauerstoff ist, z und $R^2$ H sind, und $R^1$ eine Methylgruppe ist,
daß dann $R^3$ kein 2-Pyrimidinyl-, 2-Pyridyl- oder 2-Thiazolylrest ist;
oder e) wenn
R ein Isopentylrest ist, m' 1 ist, $W^1$ und W Sauerstoff sind, n 0 ist, X Schwefel ist, und Z, $R^1$ und $R^2$ H sind,
daß dann $R^3$ kein 2-Thiazolin-2-yl-, 2-Pyridyl- oder 2-Pyrimidinylrest ist;
oder f) wenn
R ein Isopentylrest ist, m' 1 ist, $W^1$ und W Sauerstoff sind, n 0 ist, X Sauerstoff ist, Z Cl in ortho-Stellung zu $RW^1$ ist, und $R^1$ und $R^2$ H sind,
daß dann $R^3$ kein 2-Pyridyl- oder 2-Thiazolylrest ist;
oder g) wenn
m' 1 ist, X, W1 und W Sauerstoff sind, $R^3$ ein 2-Thiazolylrest ist, n 0 ist, und Z, $R^1$ und $R^2$ H sind,
daß dann R kein Isobutylrest ist.

2. Verbindung nach Anspruch 1, worin R eine $C_3$-$C_6$-Alkyl- oder $C_3$-$C_6$-Alkenylgruppe ist.

3. Verbindung nach Anspruch 1 oder 2, worin $R^3$ ein N-enthaltender Heteroring, ausgewählt aus 2-Pyridyl, 2-Pyrimidinyl, 1,3-Thiazol-2-yl ist, wobei der Heteroring unsubstituiert oder mit Halogen monosubstituiert sein kann, oder ein 2-Thiazolin-2-yl-ring ist.

4. Verbindung nach Anspruch 3, worin m' 1 ist und n 0 ist.

5. Verbindung nach Anspruch 4, worin Z Wasserstoff, W Sauerstoff, $W^1$ Sauerstoff oder Schwefel, $R^1$ Sauerstoff oder eine Methylgruppe und $R^2$ Wasserstoff, eine Methylgruppe oder Halogen ist.

6. Ungeziefer kontrollierende Zusammensetzung, umfassend eine Verbindung nach einem der Ansprüche 1 bis 5 und ein Verdünnungsmittel.

7. Verfahren zur Bekämpfung von Ungeziefer, umfassend, daß man eine das Ungeziefer bekämpfende Menge einer Verbindung nach einem der Ansprüche 1 bis 5 auf das Ungeziefer oder seinen Aufenthaltsort aufbringt.

8. Verfahren zur Herstellung von Verbindungen der Formel (A) nach einem der Ansprüche 1 bis 5, umfassend, daß man eine Verbindung der Formel (I)

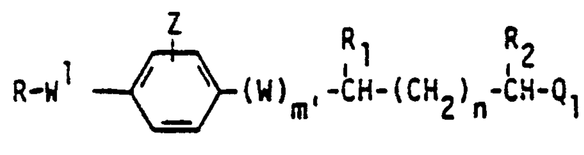

(I)

worin R, $R^1$, $R^2$, $W^1$, W, Z, m' und n wie in Anspruch 1 definiert sind, und $Q_1$ OH, SH oder eine Abgangsgruppe ist, die unter den Reaktionsbedingungen abgespalten werden kann, mit einer Verbindung der Formel (II)

$Q_2$ - $R^3$ (II)

worin $R^3$ wie in Anspruch 1 definiert ist und $Q_2$ eine Abgangsgruppe ist, die unter den Reaktionsbedingungen abgespalten werden kann, wenn $Q_1$ OH oder SH ist oder OH oder SH ist, wenn $Q_1$ eine Abgangsgruppe ist,
verethert und dann, falls erwünscht, die so erhaltenen Verbindungen der Formel (A), worin $R^2$ H, X S und W und $W^1$ 0 sind, zu Verbindungen der Formel (A), worin $R^2$ Halogen, X S und W und $W^1$ 0 sind, halogeniert.

9. Verbindung nach Anspruch 1, worin R ein 2-Butyl- oder 3-Methyl-2-butenylrest ist.

10. Verbindung nach Anspruch 1, worin R-$W^1$ $C_2H_5$-$CH(CH_3)$0, $(W)_{m'}$ 0, X-$R_3$ 0(2-Pyridyl) und $CH(R_1)$-$(CH_2)_n$-$CHR_2$ entweder $CH_2$-$CH(CH_3)$ oder $CH_2CH_2$ ist.

**Patentansprüche für folgenden Vertragsstaat : AT**

1. Ungeziefer kontrollierende Zusammensetzung, umfassend eine Verbindung der Formel (A)

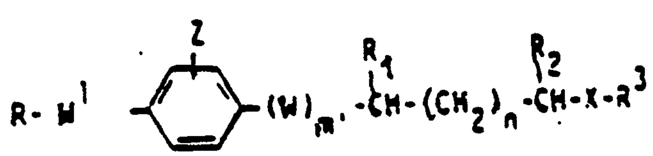

(A)

worin
m' 0 oder 1 ist;
n 0, 1 oder 2 ist;
W Sauerstoff, Schwefel, $NR^4$ oder ein Carbonylrest ist;
$W^1$ Sauerstoff, Schwefel, $NR^4$, eine Carbonyl-, Sulfinyl- oder Sulfonylgruppe ist;
X Sauerstoff oder Schwefel ist;

Z Wasserstoff, eine $C_1$-$C_8$-Alkyl-, $C_1$-$C_8$-Halogenalkylgruppe oder Halogen ist;
R eine $C_3$-$C_8$-Alkyl-, $C_2$-$C_8$-Alkenyl-, $C_2$-$C_8$-Alkinyl-, $C_1$-$C_8$-Halogenalkyl, $C_2$-$C_8$-Halogenalkenyl-, $C_2$-$C_8$-Halogenalkinyl-, $C_2$-$C_{10}$-Alkylthioalkyl-, $C_3$-$C_8$-Cycloalkyl-, $C_3$-$C_8$-Halogencycloalkyl-, $C_4$-$C_{12}$-Cycloalkylalkyl- oder Hetereocycloalkylgruppe ist;
jeder der Reste $R^1$ und $R^4$ unabhängig Wasserstoff oder ein $C_1$-$C_8$-Alkylrest ist;
$R^2$ Wasserstoff, ein $C_1$-$C_8$-Alkylrest oder Halogen ist; und entweder
$R^3$ ein aromatischer Stickstoff enthaltender Heteroring ist, ausgewählt aus Pyridyl, 3-Pyridazinyl, 2-Pyrimidinal, Pyrazinyl, Triazinyl oder 2-Thiazolyl, wobei der den Stickstoff enthaltende aromatische Heteroring unsubstituiert oder mit ein oder mehreren Substituenten, ausgewählt aus Halogen, einem $C_1$-$C_8$-Alkyl-, $C_1$-$C_8$-Halogenalkyl-, $C_1$-$C_8$-Alkoxy-, $C_1$-$C_8$-Alkylthiorest und $NO_2$, substituiert sein kann, oder eine Gruppe ($G_1$) ist:

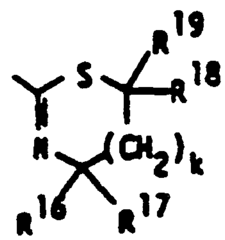

($G_1$)

worin k 0 oder 1 ist und jeder der Reste $R^{16}$, $R^{17}$, $R^{18}$ und $R^{19}$ unabhängig Wasserstoff oder ein Methylrest ist,
zusammen mit einem Verdünnungsmittel,
mit dem Vorbehalt, daß
a) wenn
m' 1 ist, X, $W^1$ und W Sauerstoff sind, $R^3$ ein 2-Pyridylrest ist, n 0 ist, und Z, $R^1$ und $R^2$ H sind,
daß dann R kein 2-Methylpropyl-, Isopentyl-, Ethyl-, n-Butyl-, n-Propyl-, n-Pentyl-, 2-Methylbutyl-, 3,3-Dimethylbutyl-, 2,2-Dimethylpropyl-, 2-Methyl-2-propenyl- oder 2-Propenylrest ist;
oder b) wenn
R ein Isopentylrest ist, m' 1 ist, $W^1$ und W Sauerstoff sind, n 0 ist, X Sauerstoff ist, und Z, $R^1$ und $R^2$ H sind,
daß dann $R^3$ kein 2-Pyrimidinyl- oder 2-Thiazolylrest ist;
oder c) wenn
R ein Isopentylrest ist, m' 1 ist, $w^1$ und W Sauerstoff sind, n 0 ist, X Sauerstoff ist, Z und $R^1$ H sind, und $R^2$ eine Methylgruppe ist,
daß dann $R^3$ kein 2-Pyridyl-, 2-Pyrimidinyl- oder 2-Thiazolylrest ist;
oder d), wenn
R ein Isopentylrest ist, m' 1 ist, $W^1$ und W Sauerstoff sind, n 0 ist, X Sauerstoff ist, Z und $R^2$ H sind, und $R^1$ eine Methylgruppe ist,
daß dann $R^3$ kein 2-Pyrimidinyl-, 2-Pyridyl- oder 2-Thiazolylrest ist;
oder e) wenn
R ein Isopentylrest ist, m' 1 ist, $W^1$ und W Sauerstoff sind, n 0 ist, X Schwefel ist, und Z, $R^1$ und $R^2$ H sind,
daß dann $R^3$ kein 2-Thiazolin-2-yl-, 2-Pyridyl- oder 2-Pyrimidinylrest ist;
oder f) wenn
R ein Isopentylrest ist, m' 1 ist, $W^1$ und W Sauerstoff sind, n 0 ist, X Sauerstoff ist, Z Cl in ortho-Stellung au $RW^1$ ist, und $R^1$und $R^2$ H sind,
daß dann $R^3$ kein 2-Pyridyl- oder 2-Thiazolylrest ist;
oder g) wenn
m' 1 ist, X, $W^1$ und W Sauerstoff sind, $R^3$ ein 2-Thiazolylrest ist, n 0 ist, und Z, $R^1$ und $R^2$ H sind,
daß dann R kein Isobutylrest ist.

2. Zusammensetzung nach Anspruch 1, worin R eine $C_3$-$C_6$-Alkyl- oder $C_3$-$C_6$-Alkenylgruppe ist.

3. Zusammensetzung nach Anspruch 1 oder 2, worin $R^3$ ein N-enthaltender Heteroring, ausgewählt aus 2-Pyridyl, 2-Pyrimidinyl, 1,3-Thiazol-2-yl ist, wobei der Heteroring unsubstituiert oder mit Halogen monosubstituiert sein kann, oder ein 2-Thiazolin-2-yl-ring ist.

4. Zusammensetzung nach Anspruch 3, worin m' 1 ist und n 0 ist.

5. Zusammensetzung nach Anspruch 4, worin Z Wasserstoff, W Sauerstoff, $W^1$ Sauerstoff oder Schwefel, $R^1$ Sauerstoff oder eine Methylgruppe und $R^2$ Wasserstoff, eine Methylgruppe oder Halogen ist.

6. Verfahren zur Bekämpfung von Ungeziefer, umfassend, daß man eine das Ungeziefer bekämpfende Menge einer Verbindung nach einem der Ansprüche 1 bis 5 auf das Ungeziefer oder seinen Aufenthaltsort aufbringt.

7. Verfahren zur Herstellung von Verbindungen der Formel (A) nach einem der Ansprüche 1 bis 5, umfassend, daß man eine Verbindung der Formel (I)

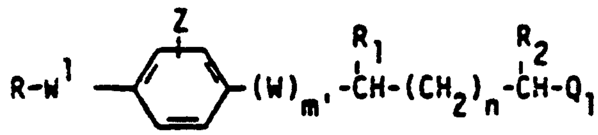 (I)

worin R, $R^1$, $R^2$, $W^1$ W, Z, m' und n wie in Anspruch 1 definiert sind, und $Q_1$ OH, SH oder eine Abgangsgruppe ist, die unter den Reaktionsbedingungen abgespalten werden kann,
mit einer Verbindung der Formel (II)

$Q_2$ - $R^3$ (II)

worin $R^3$ wie in Anspruch 1 definiert ist und $Q_2$ eine Abgangsgruppe ist, die unter den Reaktionsbedingungen abgespalten werden kann, wenn $Q_1$ OH oder SH ist oder OH oder SH ist, wenn $Q_1$ eine Abgangsgruppe ist,
verethert und dann, falls erwünscht, die so erhaltenen Verbindungen der Formel (A), worin $R^2$ H, X S und W und $W^1$ 0 sind, zu Verbindungen der Formel (A), worin $R^2$ Halogen, X S und W und $W^1$ 0 sind, halogeniert.

8. Zusammensetzung nach Anspruch 1, worin R ein 2-Butyl- oder 3-Methyl-2-butenylrest ist.

9. Zusammensetzung nach Anspruch 1, worin R-$W^1$ $C_2H_5$-$CH(CH_3)O$, (W)m' O, X-$R_3$ O-(2-Pyridyl) und $CH(R_1)$-$(CH_2)_n$-$CHR_2$ entweder $CH_2$-$CH(CH_3)$ oder $CH_2CH_2$ ist.